# EUROPEAN PATENT SPECIFICATION

(11) **EP 2 414 047 B1**
(45) Date of publication and mention of the grant of the patent: **16.01.2013**
(21) Application number: 09779255.0
(22) Date of filing: 03.04.2009
(51) Int. Cl.: A61Q 19/06, A61K 8/36, A61K 8/49, A61K 8/67, A61K 8/97

(54) **TOPICAL COSMETIC COMPOSITIONS FOR TREATING OR PREVENTING CELLULITE**
TOPISCHE KOSMETISCHE ZUSAMMENSETZUNGEN ZUR BEHANDLUNG ODER PRÄVENTION VON CELLULITITS
PREPARATIONS COSMETIQUES TOPIQUES POUR LE TRAITEMENT OU LA PREVENTION DE LA CELLULITE

(43) Date of publication of application: 08.02.2012
(73) Proprietor: Oriflame Cosmetics SA, 2340 LU Luxenbourg (LU)
(72) Inventor: AL-BADER, Tamara, S-112 51 Stockholm (SE); DAVIS, Mark, Navan (IE); LALOEUF, Aurelie, S-112 51 Stockholm (SE); RAWLINGS, A.v., Northwich Cheshire CW9 8FH (GB)
(74) Representative: Gates, Marie Christina Esther
(86) International application number: PCT/EP2009/054043
(87) International publication number: WO 2010/112084

(56) References cited:
- WO-A1-97/45100
- FR-A1- 2 847 907
- FR-A1- 2 886 546
- FR-A1- 2 916 968
- DATABASE WPI Week 199419 Thomson Scientific, London, GB; AN 1994-153280 XP002581099 & ES 2 049 661 A1 (LAB IMBEAL SA) 16 April 1994 (1994-04-16)

## Description

### Field of the Invention

The present invention relates to a topical composition that improves the aesthetic appearance of skin. In particular, it relates to a topical cosmetic composition for treating or preventing cellulite.

### Background to the Invention

Cellulite is the term applied to a skin condition associated with the lumpy uneven type of fat that is subcutaneous, producing unsightly disturbances in the skin's normally smooth curvatures. It accumulates primarily on the buttocks, thighs, and limbs of many women and may also be present on the stomach and upper arms. Although the etiology of cellulite is poorly understood, the main etiological factor appears to be local accumulation of fat in a regional compartment. It has been proposed that the anatomical structure of subcutaneous adipose tissue is the major cause of cellulite. Histological studies of subcutaneous tissues, from men and women, suggest that the fat lobules are larger and more vertical in women than men. As a result, these larger, less restricted lobules can express outward against the dermis, causing the bumps and dimples characteristic of cellulite. The tissues underlying the skin develop an 'orange peel' or 'cottage cheese' look, particularly when the skin is pinched, emphasizing the bulging and pitting of the fatty layer. The severity of cellulite tends to worsen with age and obesity, although it may also manifest itself in the skin of individuals of normal or near-normal weight. The dimpling/bumpy appearance of cellulite is a result of the deformation of the aforementioned lobules as a result of outward forces on the adipose tissue. These lobules protrude into the overlying dermis, causing a visible deformation on the surface of the skin that presents itself as cellulite.

A number of factors can be attributed to the onset of cellulite, namely, hereditary, intestinal, circulatory, lymphatic, hormonal and lifestyle factors. Fat may build up underneath the surface of the skin, due to induced lipid synthesis in the adipose layer of the skin. The buildup may take the form of a large quantity of small, but recognizable, pockets of fat underneath the surface of the skin. Its development can be further attributed to increased permeability of the capillary walls which permits the penetration of water into the connective tissue, as well as the weakening of the connective tissue and a reduction of the perfusion ratios in the blood stream and lymphatic tract. Severe cellulite is characterized by degeneration of subcutaneous blood vessels, poor blood flow, a thinning of the epidermal and dermal layers of the skin, the presence of hard lumps of fatty material surrounded by protein in the subcutaneous regions, and an accumulation and pooling of body fluids.

Dieting to decrease additional fat intake, exercise to increase fat metabolism and prevent the build up of cellulite, and massage and/or hydrotherapy may help to stimulate lymphatic drainage and reduce cellulite. However, all of these means for reducing cellulite or subcutaneous fat are limited, producing little visible results over extended periods of time. Known methodologies for cellulite treatment include localized mechanical action, topical application of chemical agents, exercise, dietary adjustments, and combinations of these therapies.

Cosmetic and dermatological compositions to ameliorate the appearance of cellulite, including natural and synthetic actives, are well known in the art. Topical application of agents capable of distributing or reducing local fat accumulation by lipolytic action (thereby improving the aesthetic appearance of the skin) have been used for the treatment of cellulite.

EP 1 827 110 describes compositions and methods for improving the appearance of skin, and in particular, treating, preventing, reducing and/or eliminating excess accumulation or production of subcutaneous fat, and also conditions related to excess production of sebum, such as acne, oily skin, hair or scalp. Treatment is achieved by topically applying compositions comprising *Alisma orientate* constituents, or extracts containing *Alisma orientate.*

EP 1 265 583 describes topical compositions containing the natural ingredient Perilla oil, which are useful to prevent, ameliorate or treat skin and other conditions, which are associated with upregulation of genes in skin cells that respond to the activation of specific receptors. The patent describes topical compositions which are useful to prevent or ameliorate the appearance of cellulite, which is associated with upregulation of Peroxisome Proliferator Activated Receptors (PPAR).

U.S. Patent No. 4,795,638 describes a cosmetic composition which can reduce or eliminate cellulite or fat build-ups on a human body, containing i) at least one rubefacient, such as capsicum extracts, nicotinic acid salts, nicotinic acid esters, and nicotinyl alcohols, and ii) at least one oil soluble plant extract (such an extract can be a mixture of ivy, rosemary, ginseng, sage, arnica, Saint John's wort, marigold, ruscus, ulmaria, orthosiphon and algae), and iii) at least one volatile organo polysiloxane.

Among the common agents for treatment of cellulite and for use as slimming agents are xanthine analogs (such as caffeine or theophylline). These agents block the antilipolytic action of adenosine, a potent endogenous inhibitor of lipolysis. A number of patents cite xanthines as substances which can be used to treat cellulite. For example, EP 1 676 606 describes a treatment for cellulite containing: caffeine, a polyurethane powder and a non-ionic dimethione copolyol. U.S. Patent No. 5,536,499 describes compositions for increasing the strength and firmness of the skin and reducing the signs of cellulite. The compositions described contain inositol phosphate and caffeine.

EP 1 171 089 describes topical compositions for regulating the condition of the skin including the treatment of cellulite. The compositions described therein include, among other ingredients, a retinoid. Retinoids are well known in the art as substances which can be used to improve the appearance of the skin. The group retinoids includes: retinol, retinol esters, retinal and/or retinoic acid (including all-trans retinoic acids and/or 13-cis-retinoic acid). Other patents relating to the use of retinoids in skincare compositions include EP 1 514 536 and EP 0 631 772, both from Johnson and Johnson.

EP 1 214 048, which is owned by Pentapharm, relates to the use of conjugated linoleic acid (CLA) for the topical treatment of fatty deposits and cellulite. It describes cosmetic and dermatological topical compositions for the treatment of fatty deposits and cellulite comprising CLA. REGU^{®}-SHAPE is a commercially available material (Pentapharm) which contains CLA and claims to be able to decrease the accumulation of new lipids and stimulate the breakdown of new lipids in the adipocytes.

International Publication No. WO 2007/077541 of Sederma, concerns the use of glaucine or a plant extract containing it, as an active ingredient, used alone or in association with at least another cosmetic/dermatological active, for the preparation of a topical composition to treat cellulite by inducing adipocyte reversion. Ovaliss^{™} is a commercially available product (Sederma) which contains glaucine, and which has been shown to reduce thickness of fatty tissue, particularly in treating double chin fat and for visibly reshaping facial contours.

Despite the efforts made in the prior art to prepare topical anti-cellulite preparations, there remains a need for improved highly efficacious, safe and natural compositions to treat, prevent, reduce, inhibit, and/or improve the signs of excess accumulation and production of subcutaneous fat.

It is therefore an object of the invention to provide improved topical compositions that improve the aesthetic appearance of skin. In particular, it is an object of the invention to provide improved topical cosmetic compositions that can break down the build-up of subcutaneous fat and thus reduce, improve or prevent the formation and development of cellulite in human skin.

### Summary of the Invention

Accordingly, the invention provides a topical composition for treating and/or preventing cellulite, said composition comprising:
(i) a therapeutically effective amount of each of *Furcellaria lumbricalis* and *Fucus vesiculosus;*
(ii) a therapeutically effective amount of one or more skin care actives selected from the group consisting of: glaucine, retinol, conjugated linoleic acid and caffeine; and
(iii) a dermatologically acceptable carrier.

The composition suitably comprises *Furcellaria lumbricalis* in an amount in the range from 0.001% to 25%, preferably in the range 0.005% to 5%.

The composition suitably comprises *Fucus vesiculosus* in an amount in the range from 0.001 % to 25%, preferably in the range 0.004% to 5%.

The cosmetic composition according to the invention comprises *Furcellaria lumbricalis* and *Fucus vesiculosus,* which are collectively termed the "Baltic Algaes", for the purposes of the present invention.

*Furcellaria Lumbricalis* is a red seaweed/algae found along the coast of the Baltic Sea. It is not known to be used in cosmetic preparations and hitherto, this ingredient has not been reported to exhibit any activity in the breakdown of fat in adipose tissue.

*Fucus Vesiculosus* is a seaweed/algae found on the coasts of the North Sea, the western Baltic Sea, and the Atlantic. It is known to have slimming properties and is used in a number of anti-cellulite products on the market.

The inventors have found that the Baltic Algaes, when used together with one or more skin care actives such as glaucine, retinol, conjugated linoleic acid or caffeine, act synergistically in the breaking down of fat in human adipose tissue *in vitro.* The surprising results demonstrate more than a mere additive effect.

In one embodiment, the composition comprises glaucine in an amount in the range 0.0001% to 25%, preferably in the range 0.002% to 5%.

In an alternative embodiment the composition further comprises retinol in an amount in the range 0.0001% to 5%, preferably in the range 0.01% to 2%.

In a further alternative embodiment the composition may comprise conjugated linoleic acid in an amount in the range from 0.01% to 25%, preferably in the range from 0.1% to 5%.

In a still further embodiment, the composition suitably comprises caffeine in an amount in the range from 0.001% to 2%, preferably in the range 0.05% to 0.5%.

In one embodiment, the invention provides a topical anti-cellulite composition comprising a therapeutically effective amount of a combination of *Furcellaria lumbricalis* and *Fucus vesiculosus,* together with a therapeutically effective amount of glaucine. The composition according to this embodiment suitably comprises:

| | wt % |
|---|---|
| (i) *Furcellaria lumbricalis* | 0.005 - 5 |
| (ii) *Fucus vesiculosus* | 0.004 - 5 |
| (iii) Glaucine | 0.002 - 5 |
| and | |
| (iv) a dermatologically acceptable carrier | to 100. |

In an alternative embodiment, the composition comprises:

| | wt% |
|---|---|
| (i) *Funcellaria lumbricalis* | 0.005 - 5 |
| (ii) *Fucus vesiculosus* | 0.004 - 5 |
| (iii) Retinol | 0.01 - 2 |
| (iv) Conjugated Linoleic Acid | 0.1 - 5 |
| and | |
| (v) a dermatologically acceptable carrier | to 100. |

In a further alternative embodiment, the composition comprises:

| | wt % |
|---|---|
| (i) *Furcellaria lumbricalis* | 0.005 - 5 |
| (ii) *Fucus vesiculosus* | 0.004 - 5 |
| (iii) Glaucine | 0.002 - 5 |
| (iv) Retinol | 0.01 - 2 |
| (v) Conjugated Linoleic Acid | 0.1 - 5 |
| and | |
| (vi) a dermatologically acceptable carrier | to 100. |

The dermatologically acceptable carrier may be selected from the group consisting of water, liquid or solid emollients, humectants and solvents.

The carrier will usually form between 5% to 99.9% (w/w), preferably from 25% to 90% by weight of the composition and can, in the absence of other cosmetic adjuncts, form the balance of the composition.

The composition may further comprise ingredients selected from the group consisting of silicones, emulsifiers, thickeners, powders, film formers, rheology modifying agents and propellants.

Suitably, the composition further comprises one or more agents selected from the group consisting of fatty acids, moisturising agents and delivery enhancers.

The one or more fatty acids may be selected from the group consisting of stearic acid, palmitic acid, lauric acid, myristoleic acid, palmitoleic acid, oleic acid, linoleic acid, linolenic acid, arachidonic acid, eicosapentaenoic acid, erucic acid and docosahexaenoic acid.

The one or more moisturising agents may be selected from the group consisting of glycerine, sorbitol, trehalose, urea, butylene glycol, propylene glycol, pentylene glycol and betaine.

The composition may further comprise one or more delivery enhancers selected from the group consisting of isohexadecane, diisopropyl dimer dilinoleate, dimethicone, cyclopentasiloxane and cyclohexasiloxane.

The composition may be formulated to improve the delivery of the actives to the skin. This is based on the assumption that the delivery of certain actives, such as retinol, for example, can be enhanced by making the active less soluble in the formulation, than in the skin itself. Delivery enhancers, such as those mentioned above, are not particularly good solvents for retinol in comparison with the skin. Therefore, when the formulation is applied to the skin the retinol diffuses into the skin where it is more soluble.

The composition may further comprise adjuncts such as fragrance, opacifiers, preservatives, colourants, buffers, mattifying agents and other anti-aging ingredients.

The topical composition according to the present invention may be prepared in a manner well known in the art of preparing skin care products. The active components are generally incorporated in a dermatologically acceptable carrier. The active components can suitably first be dissolved or dispersed in a portion of the water or another solvent or liquid to be incorporated in the composition.

The preferred compositions are oil-in-water or water-in-oil emulsions.

The composition according to the invention may be in the form of a cream, lotion, ointment, milk, gel. The composition can also be in the form of a cleanser such as a facial cleanser, exfoliator, peel, body scrub, a shampoo, a shower or bath gel. The composition may also be used as a progressive body tanner or progressive face tanner with slimming action. The composition may also be used as a body mask, body cocoon or body wrap. This involves applying the composition according to the invention to the skin as a thick substantial formula (for example, a cream, lotion, mud or clay), then wrapping the body or body part in bandages or cloth for a period of time (for example, twenty minutes) to keep warm. The composition may also be in the form of a foundation or other colour product.

Most preferably the product is a "leave-on'' topical product, that is, a product to be applied to the skin without a deliberate rinsing step soon after its application to the skin.

The composition may further comprise a therapeutically effective amount of anti-ageing actives. Suitably, the composition further comprises hydroxy acids. Exemplary actives suitable for use in the compositions of the present invention include alpha-hydroxy acids, such as lactic acid or glycolic acid; or beta-hydroxy acids such as, salicylic acid or salicylic acid derivatives; or vitamins or vitamin derivatives.

Compositions according to the invention may further comprise naturally occurring or synthesized peptides including di-, tri-, tetra-, pentapeptides and/or derivatives thereof. For example, the composition may further comprise a tripeptide of sequence Arg-Gly-Ser. This tripeptide composition, which is described in Irish Patent No. S84694, is known to have a photo-protecting and regenerating effect on the skin.

The compositions according the invention may further comprise UV filters. Suitable UV filters include ethylhexyl methoxycinnamate, octocrylene, butyl methoxydibenzoylmethane, ethylhexyl salicylate, titanium dioxide, zinc oxide, or methylene bis-benzotriazolyl tetramethylbutylphenol, for example. Tanning agents such as dihydroxy acetone, for example, may also be incorporated in the compositions according to the invention.

The composition may further comprise an effective amount of amino acids that are known to play a role in fatty acid metabolism. Such amino acids include L-carnitine, for example.

Actives such as natural plant extracts, for example, those that demonstrate PPAR-ligand activity or anti-inflammatory activity, may also be included in the composition according to the invention. Suitable plant extracts are those containing flavonoids, isoprenols and/or tripterpene acids, which exhibit PPAR-ligand activities; such extracts include, but are not limited to: ursolic acid, gallic acid, oleanolic acid, ellagic acid, rosmarinic acid or asiatic acid, and derivatives thereof. Plants, herbs and spices may be used for developing these extracts (for use herein) and include, but are not limited to, pomegranate flower, seed and/or bark extract, liquorice extract, gingseng extract and/or chilli extracts.

The composition may be packaged in any suitable manner such as a jar, a bottle, a tube, a pump, a roll-ball, a stick or sachet. It may also be incorporated into a fabric, such as a wipe, glove or mitten, for example.

The invention further provides a cosmetic method of treating or preventing skin conditions such as cellulite, the method comprising applying to the skin the topical composition as described herein. The amount of the composition which is applied, the frequency of application and the period of use will vary widely depending upon the body area that is being treated. For treatment of cellulite in the thigh and buttock regions, which are particularly prone to agglomerations of fat and water, the composition can typically be applied twice to three times daily, followed by massaging into the skin to increase the absorption of the formulation into the skin and stimulate lymphatic drainage. A light, circular massage motion, which may be achieved with the aid of a glove, mitten or other apparatus, should be used until all the composition has been absorbed into the skin. Best results are achieved by gentle exfoliation of the skin or showering before applying the product.

The invention further provides for the use of a therapeutically effective amount of each of:
(i) *Furcellaria lumbricalis* and
(ii) *Fucus vesiculosus,* together with
(iii) a therapeutically effective amount of one or more skin care actives selected from the group consisting of: glaucine, retinol, conjugated linoleic acid and caffeine; and
(iv) a dermatologically acceptable carrier, in the manufacture of a cosmetic composition for treating cellulite.

The cosmetic composition is particularly suitable for use in the treatment of conditions such as cellulite and sagging skin.

The term "therapeutically effective amount", as used herein, means an amount effective to break down fat in human adipose tissue and thereby preventing and/or treating cellulite.

The term "treating", as used herein, includes within its scope reducing, delaying and/or preventing the appearance of cellulite, and additionally, generally enhancing the quality of skin by improving its appearance and texture. The topical compositions according to the invention may be useful for treating skin which is already suffering from cellulite or to treat youthful skin to prevent or reduce the appearance of cellulite.

The term "dermatologically acceptable carrier", as used herein, means that the carriers described are suitable for use in contact with mammalian keratinous tissue without causing any adverse effects such as undue toxicity, incompatibility, instability, allergic response, for example.

The present invention describes novel compositions for the treatment of cellulite. The embodiments, objectives and advantages or the present invention will become apparent in the following description to a person who is skilled in the art.

### Brief Description of the Drawings

Figure 1 shows the lipolytic effect of Baltic algae and Ovaliss^{™} (glaucine) on the lipolytic effect of mature adipocytes;
Figure 2 shows the lipolytic effect of Baltic algae, Retinol and Conjugated linoleic acid on mature adipocytes; and
Figure 3 shows the lipolytic effect of Baltic algae, Retinol, Conjugated linoleic acid and Ovaliss^{™} (glaucine) on mature adipocytes.

### Detailed Description of the Invention

The invention provides a topical cosmetic composition for the treatment and/or prevention of cellulite. The combination of actives in the composition surprisingly demonstrates a synergistic effect on the breakdown of fat cells in human adipose tissue.

The mechanism of action of anti-cellulite agents is as follows: adipocytes are cells found in adipose tissue, that function as energy stores by reserving the body's excess energy in the form of triacylglycerol. Adipocytes, thereby play a specialized role in energy metabolism. Cell precursors of adipocytes, pre-adipocytes, can differentiate into mature adipocytes when influenced by a variety of stimuli, leading to their characteristic accumulation of fatty deposits (triglycerides) within the cells. During this process, the cells convert from a fibroblastic shape to a spherical shape, and dramatic changes occur in the cell morphology, cytoskeletal components, and the level and types of extracellular matrix components (ECM). Adipocyte differentiation also involves communication of extracellular signals and those of the ECM environment to the nucleus. Transcription factors play an important role in adipocyte differentiation. The peroxisome proliferator-activated receptor (PPAR) family of transcription factors are induced early during adipocyte differentiation, with PPAR-γ being largely adipocyte specific, with low but detectable levels expressed in pre-adipocytes, and maximum expression being in mature adipocytes. During the end stages of cell differentiation, lipogenesis mechanisms are strongly activated, resulting in the production of triglycerides within the adipocytes.

Two receptors on the outer membrane surface of adipocytes are believed to control the fat volume of the cell. These receptors are a β receptor, activation of which stimulates lipolysis and an α₂ receptor, activation of which stimulates lipogenesis and inhibits lipolysis. Lipolysis is a process whereby the triglycerides within the adipocytes are broken down. This is, therefore, a very desirable mechanism to stimulate in a cellulite treatment. The best known mechanism for mediating lipolysis is the cLAMP pathway. Here, coupling of the hormone receptors in the plasma membrane to a G-family of GTP-binding protein occurs. The alpha subunit of the G-protein stimulates adenylyl cyclise, which produces cAMP. The resulting increase in intracellular cAMP levels leads to the activation of a cAMP dependent protein kinase, PKA. Hormone sensitive lipase (HSL) and perilipin are phosphorylated by PKA and this leads to catalysis of triglycerides breakdown, producing the subsequent release of free fatty acids and glycerol (Gonzalez-Yanes Carmen et al., Cellular Signalling 18:401-8 2006).

Normally there is an equilibrium between lipogenesis and lipolysis, however, if an imbalance arises between these mechanisms, triglycerides accumulate within the adipocytes. According to the receptor models, the treatment for cellulite should look towards stimulating the β receptor, β-adrenergic stimulation, while the α₂ receptor should be inhibited (α₂ -adrenergic inhibition).

The present invention provides a cosmetic composition which comprises a novel combination of key ingredients which have been shown by the inventor to surprisingly act synergistically in the breaking down of fat in human adipose tissue *in vitro.* The key ingredients are *Furcellaria lumbricalis, Fucus vesiculosus,* glaucine, retinoids and conjugated linoleic acid.

*Furcellaria lumbricalis* is a red seaweed/algae found along the coast of the Baltic Sea. It is not known to be used in cosmetic anti-cellulite preparations. Extracts of *Furcellaria lumbricadis* contain a high number of polysaccharides, the major components being sulphated galactans, commercially known as furcellaran and carrageenans, and has been widely used in food industry due to its unique properties, such as gelation, emulsification and protein stabilization

*Fucus vesiculosus,* commonly known as bladder wrack, is a seaweed/algae found on the coasts of the North Sea, the western Baltic Sea, and the Atlantic. It has a number of chemical constituents namely, mucilage, algin, mannitol, beta-carotene, zeaxanthin, naturally occuring glycosaminoglycans (such as fucans and fucoidans) and many other minerals. Fucus vesiculosus is known to having slimming properties and has been used in prior art compositions for the treatment of cellulite. However, the present inventors have found that, surprisingly, when these "Baltic Algaes" *(Furcellaria lumbricalis* and *Fucus vesiculosus)* are combined with one or more anti-cellulite agents, for example, glaucine, retinol and/or CLA, a synergistic effect on the breakdown of fat in adipose tissue is observed.

Glaucine is an alkaloid found in several different plant species such as Glaucium flavum, Glaucium oxylobum, Croton lechleri and Corydalis yanhusuo, for example. The glaucine used in the compositions of the present invention is contained in a product called Ovaliss™, which is a glycerine-based emulsion and is available from the supplier, Sederma. Ovaliss™ has been shown to cause the breakdown of fat in human adipose tissue.

The compositions of the present invention may also contain a retinoid, which includes all natural and/or synthetic analogs of Vitamin A or retinol-like compounds which possess the biological activity of Vitamin A. The retinoid used is preferably retinol. This compound is well known in the art and is commercially available from a number of sources, e.g., RetiSTAR^{®} from BASF.

Conjugated linoleic acid (CLA) is a mixture of positional and configurational isomers of octadecadienoic acid, which are naturally occurring substances found in milk and dairy products as well as in meats of ruminants. CLA is also known as a slimming agent and has been used to prepare topical treatments for the prevention of fatty deposits and cellulite. REGU^{®}-SHAPE is a commercially available product from Pentapharm which contains CLA.

### EXPERIMENTAL METHOD

### Pre-adipocyte Culture & Maturation into Adipocytes

### Human pre-adipocytes were purchased from Zen-Bio (USA).

Pre-adipocytes were plated into wells of a 96 well plate, in Preadipocyte Medium (cat: PM-1) and allowed to differentiate for one week. After this time, the entire media volume is aspirated from the adherent pre-adipocytes and Adipocyte Differentiation Medium (cat: DM-2) is added to each of the wells. These cells are left to culture for 7 days at 37°C and 5% CO₂. After 7 days the cells are fed by taking out the media and replacing with fresh Adipocyte Medium (cat: AM-1) and leaving to culture for another 7 days at 37°C and 5% CO₂. Three weeks after the initiation of differentiation the cells appear rounded containing many intracellular lipid droplets and are then considered as mature adipocytes.

### Active Test Compounds

• Baltic Algae (Codif): *Furcellania lumbnicalis 0.1 %*
   *Fucus vesiculosus* 0.1%
• Retinol (Sigma): 0.5µM
• Ovaliss (Sederma): 0.1%
• CLA (Pentapharm): 0.003%

Concentrations chosen for treatment are based on dose response experiments where the toxicity levels were determined.

### Treatment of Adipocytes with Actives

A Lipolysis Assay kit (cat: LIP-1 or LIP-NC) from Zen-Bio was used to measure the release of free glycerol by the mature adipocytes following treatment. Active test compounds were made up to the appropriate concentrations in Assay Buffer. To test for synergy, various test compounds were combined into the assay buffer at the concentration indicated, for treatment on the mature adipocytes. Positive control treatment, Isoproterenol, provided in the kit was included as a separate treatment. Negative control treatments consisted of 0.1% DMSO (as this is the carrier for the Isoproterenol).

Prior to the adipocyte treatment with test compounds, the cells were gently washed in wash buffer (provided in the kit) twice, after which the wash buffer was discarded. Adipocytes were subsequently treated with 100µl of active test compounds (either single actives or in combination to determine synergy), positive or negative controls for 18hr at 37°C and 5% CO₂. Cells were treated in triplicate per experiment.

The glycerol detection assay (from the Lipolysis kit) was set up an hour prior to the commencement of the assay. Here dose dependent glycerol standards were prepared together with Glycerol detection reagents, as directed by the manufacturer. Following treatment of adipocytes with test compounds, 50µl of the cell media is removed from each of the treated wells and transferred into a fresh 96-well plate, in triplicate. 50µl of glycerol standards, positive & negative controls were also added to this plate, in triplicate. Glycerol detection reagent was then added to each of these wells and the plate was incubated for 15min at room temperature.

Colour change is determined by measuring the optical density of each well at 540nm. The amount of glycerol in each of the treatment wells is then determined by extrapolating from the glycerol standard curve.

Fat from mature adipocytes breaks down into glycerol and other compounds when the cells are exposed to lipolytic agents. The glycerol detection assay allows for the measurement of glycerol that was released from the adipocytes after treatment with test compounds. The higher the concentration of glycerol detected, the more lipolysis has occurred. In the analysis of the results, the background glycerol production level in the negative control is deducted from the treatment conditions with test compounds.

### EXPERIMENTAL SYNERGY RESULTS

### Example 1

### Lipolytic effect of Baltic algae and/or Ovaliss^{™} (glaucine) on the lipolytic effect of mature adipocytes.

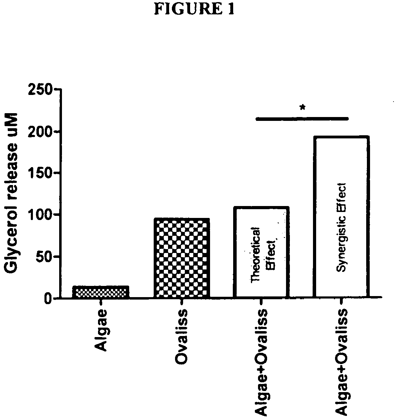

Using the methods described above to measure the amount of glycerol released from mature adipocytes as an indication of fat breakdown in the cells, the inventor has surprisingly found that the combination of the Baltic algae and Ovaliss^{™} (glaucine) act synergistically to result in greater glycerol release than the theoretically added values of the Algae or Ovaliss^{™} (glaucine) treatment alone (Figure 1). This synergistic effect was found to be statistically significant (* p < 0.05, student's t test).

### Example 2

### Lipolytic effect of Baltic algae and/or Retinol and Conjugated linoleic acid on mature adipocytes.

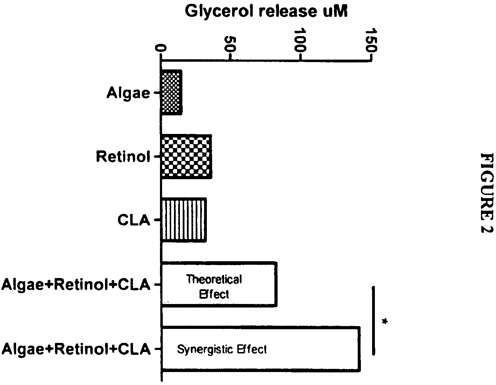

The lipolytic effect of Baltic algae and/or retinol and conjugated linoleic acid on mature adipocytes was investigated using the method set out above. The results are shown in Figure 2. The combination of Baltic Algae with retinol and conjugated linoleic acid on mature adipocytes also resulted in the synergistic release of glycerol, compared to the theoretically added individual values (* p < 0.05, student's t test).

### Example 3

### Lipolytic effect of Baltic algae, Retinol, Conjugated linoleic acid and Ovaliss^{™} (glaucine) on mature adipocytes.

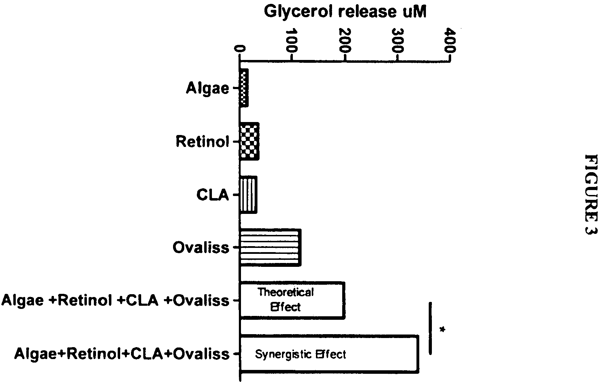

The lipolytic effect of Baltic algae, Retinol, Conjugated linoleic acid and Ovaliss^{™} (glaucine) on mature adipocytes was investigated following the method set out above. The results are shown in Figure 3. The combination of Baltic Algae with retinol, Conjugated linoleic acid and Ovaliss^{™} (glaucine) on mature adipocytes surprisingly resulted in the synergistic release of glycerol, compared to the theoretical additive values of the individual treatments (* p significant at 0.05 level, student's t test).

### Example 4

The formulation below describes an oil-in-water cream incorporating the inventive composition which is suitable for the methods and uses according to the present invention. The percentages indicated are by weight of the composition unless stated otherwise.

| Component Description | Weight % |
|---|---|
| AQUA | to 100 |
| BUTYLENE GLYCOL | 10 |
| ISOHEXADECANE | 5 |
| GLYCERIN | 4.6 |
| DIMETHICONE | 3 |
| CYCLOPENTA\HEXASILOXANE | 3 |
| DIISOPROPYL DIMER DILINOLEATE | 2 |
| GLYCERYL STEARATE\PEG-100 STEARATE | 2.5 |
| CETYL ALCOHOL | 1 |
| STEARIC ACID | 1 |
| BUTYROSPERMUM PARKII BUTTER | 0.5 |
| CAPRYLIC/CAPRIC TRIGLYCERIDE | 0.5 |
| ACRYLATES/C10-30 ALKYL ACRYLATE CROSSPOLYMER | 0.2 |
| ISOMERIZED LINOLEIC ACID | 0.2 |
| SODIUM ASCORBATE | 0.2 |
| TOCOPHEROL | 0.2 |
| CAFFEINE | 0.1 |
| RETINOL | 0.05 |
| FUCUS VESICULOSUS EXTRACT | 0.03 |
| CAPRYLYL GLYCOL | 0.03 |
| FURCELLARIA LUMBRICALIS EXTRACT | 0.005 |
| GLAUCINE | 0.002 |
| PRESERVATIVES | QS |
| FRAGRANCE | QS |
| BHT | QS |
| DISODIUM EDTA | QS |
| SODIUM HYDROXIDE | QS |

### Example 5

The formulation below describes an oil-in-water cream incorporating the inventive composition which is suitable for the methods and uses according to the present invention. The percentages indicated are by weight of the composition unless stated otherwise.

| COMPONENT DESCRIPTION | WEIGHT % |
|---|---|
| AQUA | To 100 |
| BUTYLENE GLYCOL | 10 |
| ISOHEXADECANE | 5 |
| GLYCERIN | 4.5 |
| DIMETHICONE | 3 |
| CYCLOPENTA\HEXASILOXANE | 3 |
| DIISOPROPYL DIMER DILINOLEATE | 2 |
| GLYCERYL STEARATE\PEG-100 STEARATE | 2.5 |
| CETYL ALCOHOL | 1 |
| STEARIC ACID | 1 |
| HYDROXYETHYL ACRYLATE/SODIUM ACRYLOYLDIMETHYL TAURATE COPOLYMER | 1 |
| BUTYROSPERMUM PARKII BUTTER | 0.5 |
| POLYISOBUTENE | 0.3 |
| ISOMERIZED LINOLEIC ACID MIXTURE | 0.5 |
| PEG-7 TRIMETHYLOLPROPANE COCONUT ETHER | 0.05 |
| CAFFEINE | 0.1 |
| FUCUS VESICULOSUS EXTRACT | 1 |
| FURCELLARIA LUMBRICALIS EXTRACT | 1 |
| GLAUCINE EXTRACT | 2 |
| RETINOL | 0.05 |
| PRESERVATIVES | QS |
| FRAGRANCE | QS |
| SODIUM HYDROXIDE | QS |

### Example 6

The formulation below describes an oil-in-water cream incorporating the inventive composition which is suitable for the methods and uses according to the present invention. The percentages indicated are by weight of the composition unless stated otherwise.

| Component Description | Weight % |
|---|---|
| AQUA | to 100 |
| BUTYLENE GLYCOL | 10 |
| ISOHEXADECANE | 5 |
| GLYCERIN | 4.5 |
| DIMETHICONE | 3 |
| CYCLOPENTA\HEXASILOXANE | 3 |
| DIISOPROPYL DIMER DILINOLEATE | 2 |
| STEARETH-2 | 3 |
| STEARETH-21 | 1 |
| CETYL ALCOHOL | 1 |
| STEARIC ACID | 1 |
| BUTYROSPERMUM PARKII BUTTER | 0.5 |
| CAPRYLIC/CAPRIC TRIGLYCERIDE | 0.5 |
| ACRYLATES/C10-30 ALKYL ACRYLATE CROSSPOLYMER | 0.2 |
| ISOMERIZED LINOLEIC ACID MIXTURE | 0.5 |
| CAFFEINE | 0.1 |
| RETINOL | 0.05 |
| FUCUS VESICULOSUS EXTRACT | 1 |
| FURCELLARIA LUMBRICALIS EXTRACT | 1 |
| GLAUCINE EXTRACT | 2 |
| PRESERVATIVES | QS |
| FRAGRANCE | QS |
| SODIUM HYDROXIDE | QS |

### Example 7

The formulation below describes a water-in-oil anti-cellulite cream incorporating the inventive composition which is suitable for the methods and uses according to the present invention. The percentages indicated are by weight of the composition unless stated otherwise.

| Component Description | Weight % |
|---|---|
| AQUA | to 100 |
| BUTYLENE GLYCOL | 10 |
| ISOHEXADECANE | 15 |
| GLYCERIN | 4.5 |
| DIMETHICONE | 3 |
| CYCLOPENTA\HEXASILOXANE | 4 |
| DIISOPROPYL DIMER DILINOLEATE | 10 |
| PEG-10 DIMETHICONE | 1.5 |
| MAGNESIUM SULFATE | 0.75 |
| DISTEARDIMONIUM HECTORITE | 0.4 |
| CAPRYLIC/CAPRIC TRIGLYCERIDE | 0.5 |
| ISOMERIZED LINOLEIC ACID MIXTURE | 0.5 |
| CAFFEINE | 0.1 |
| RETINOL | 0.05 |
| FUCUS VESICULOSUS EXTRACT | 1 |
| FURCELLARIA LUMBRICALIS EXTRACT | 1 |
| GLAUCINE EXTRACT | 2 |
| PRESERVATIVES | QS |
| FRAGRANCE | QS |
| SODIUM HYDROXIDE | QS |

### Example 8

The formulation below describes a water-in-silicone cream for face, neck, eyes, décolleté or body, incorporating the inventive composition which is suitable for the methods and uses according to the present invention. The percentages indicated are by weight of the composition unless stated otherwise.

| Component Description | Weight Share |
|---|---|
| AQUA | To 100 |
| CYCLOPENTA\HEXASILOXANE | 23 |
| DIMETHICONE | 3 |
| POLYGLYCERYL-4 ISOSTEARATE | 2 |
| BUTYLENE GLYCOL | 5 |
| CETYL PEG/PPG-10/1 DIMETHICONE | 2 |
| HEXYL LAURATE | 2 |
| BIS-PEG/PPG-14/14 DIMETHICONE | 1.3 |
| DISTEARDIMONIUM HECTORITE | 1 |
| SODIUM CHLORIDE | 1 |
| CAFFEINE | 10.1 |
| RETINOL | 0.05 |
| FURCELLARIA LUMBRICALIS EXTRACT | 1 |
| FUCUS VESICULOSUS EXTRACT | 1 |
| GLAUCINE EXTRACT | 2 |
| ISOMERISED LINOLEIC ACID | 0.5 |
| FRAGRANCE | QS |
| PRESERVATIVES | QS |
| SODIUM HYDROXIDE | QS |

### Example 9

The formulation below describes a silicone-in-water cream for face, neck, eyes, décolleté or body, incorporating the inventive composition which is suitable for the methods and uses according to the present invention. The percentages indicated are by weight of the composition unless stated otherwise.

| Component Description | Weight Share |
|---|---|
| AQUA | To 100 |
| GLYCERIN | 3 |
| DIMETHICONE | 3 |
| BETAINE | 2 |
| BUTYLENE GLYCOL | 10 |
| PEG-40 HYDROGENATED CASTOR OIL | 1.5 |
| SODIUM ACRYLATE/ACRYLOYLDIMETHYL TAURATE COPOLYMER | 0.4 |
| ISOHEXADECANE | 0.2 |
| ACRYLATES/C10-30 ALKYL ACRYLATE CROSSPOLYMER | 0.2 |
| DIMETHICONOL | 0.1 |
| POLYSORBATE 80 | 0.1 |
| RETINOL | 0.05 |
| CAFFEINE | 0.1 |
| GLAUCINE EXTRACT | 2 |
| FURCELARIA LUMBRICALIS EXTRACT | 1 |
| FUCUS VESICULOSUS EXTRACT | 1 |
| ISOMERISED LINOLEIC ACID | 0.5 |
| FRAGRANCE | QS |
| PRESERVATIVES | QS |
| SODIUM HYDROXIDE | QS |

### Example 10

The formulation below describes a water-in-oil foundation, concealer or lip cream incorporating the inventive composition which is suitable for the methods and uses according to the present invention. The percentages indicated are by weight of the composition unless stated otherwise.

| Component Description | Weight Share |
|---|---|
| AQUA | To 100 |
| BUTYLENE GLYCOL | 10 |
| DICAPRYLYL ETHER | 8.5 |
| DICAPRYLYL CARBONATE | 6 |
| TITANIUM DIOXIDE | 4.9 |
| GLYCERIN | 3 |
| POLYGLYCERYL-3 DIISOSTEARATE | 2.5 |
| SUCROSE POLYSTEARATE | 1.5 |
| CI 77492 | 1.4 |
| DIMETHICONE | 1 |
| METHYL METHACRYLATE CROSSPOLYMER | 1 |
| TOURMALINA | 1 |
| MAGNESIUM SULFATE | 0.5 |
| CI 77491 | 0.3 |
| GLYCERYL DIBEHENATE | 0.2 |
| TRIBEHENIN | 0.15 |
| CI 77499 | 0.1 |
| FURCELLARIA LUMBRICALIS EXTRACT | 1 |
| RETINOL | 0.05 |
| FUCUS VESICULOSUS EXTRACT | 1 |
| ISOMERISED LINOLEIC ACID | 0.5 |
| CAFFEINE | 0.1 |
| FRAGRANCE | QS |
| PRESERVATIVE | QS |
| SODIUM HYDROXIDE | QS |

### Example 11

The formulation below describes a water/oil/water (W/O/W) triple emulsion suitable for body, face, neck, eyes, decollete, incorporating the inventive composition which is suitable for the methods and uses according to the present invention. The percentages indicated are by weight of the composition unless stated otherwise.

| Component Description | Weight Share |
|---|---|
| AQUA | To 100 |
| BUTYLENE GLYCOL | 10 |
| ISOHEXADECANE | 10 |
| DIISOPROPYL DIMER DILINOLEATE | 5 |
| GLYCERIN | 4 |
| CETYL PEG/PPG-10/1 DIMETHICONE | 2 |
| COCO-GLUCOSIDE | 0.6 |
| SODIUM CHLORIDE | 0.5 |
| CAPRYLIC/CAPRIC TRIGLYCERIDE | 0.5 |
| ACRYLATES/C10-30 ALKYL ACRYLATE CROSSPOLYMER | 0.2 |
| XANTHAN GUM | 0.1 |
| CAFFEINE | 0.1 |
| RETINOL | 0.05 |
| FUCUS VESICULOSUS EXTRACT | 1 |
| FURCELLARIA LUMBRICALIS EXTRACT | 1 |
| GLAUCINE EXTRACT | 2 |
| ISOMERISED LINOLEIC ACID | 0.5 |
| PRESERVATIVES | QS |
| FRAGRANCE | QS |
| SODIUM HYDROXIDE | QS |

### Example 12

The formulation below describes an oil-in-water cream incorporating the inventive composition which is suitable for the methods and uses according to the present invention. The percentages indicated are by weight of the composition unless stated otherwise. This example of the invention does not contain glaucine extract.

| Component Description | Weight % |
|---|---|
| AQUA | to 100 |
| BUTYLENE GLYCOL | 10 |
| ISOHEXADECANE | 5 |
| GLYCERIN | 4.5 |
| DIMETHICONE | 3 |
| CYCLOPENTA\HEXASILOXANE | 3 |
| DIISOPROPYL DIMER DILINOLEATE | 2 |
| GLYCERYL STEARATE\PEG-100 STEARATE | 2.5 |
| CETYL ALCOHOL | 1 |
| STEARIC ACID | 1 |
| BUTYROSPERMUM PARKII BUTTER | 0.5 |
| CAPRYLIC/CAPRIC TRIGLYCERIDE | 0.5 |
| ACRYLATES/C10-30 ALKYL ACRYLATE CROSSPOLYMER | 0.2 |
| ISOMERIZED LINOLEIC ACID MIXTURE | 0.5 |
| CAFFEINE | 0.1 |
| RETINOL | 0.05 |
| FUCUS VESICULOSUS EXTRACT | 0.8 |
| FURCELLARIA LUMBRICALIS EXTRACT | 1 |
| PRESERVATIVES | QS |
| FRAGRANCE | QS |
| SODIUM HYDROXIDE | QS |

### Example 13

The formulation below describes an oil-in-water cream incorporating the inventive composition which is suitable for the methods and uses according to the present invention. The percentages indicated are by weight of the composition unless stated otherwise. This example of the invention does not contain glaucine extract.

| COMPONENT DESCRIPTION | WEIGHT % |
|---|---|
| AQUA | To 100 |
| BUTYLENE GLYCOL | 10 |
| ISOHEXADECANE | 5 |
| GLYCERIN | 4.5 |
| DIMETHICONE | 3 |
| CYCLOPENTA\HEXASILOXANE | 3 |
| DIISOPROPYL DIMER DILINOLEATE | 2 |
| GLYCERYL STEARATE\PEG-100 STEARATE | 2.5 |
| CETYL ALCOHOL | 1 |
| STEARIC ACID | 1 |
| HYDROXYETHYL ACRYLATE/SODIUM ACRYLOYLDIMETHYL TAURATE COPOLYMER | 1 |
| BUTYROSPERMUM PARKII BUTTER | 0.5 |
| ISOMERIZED LINOLEIC ACID MIXTURE | 0.5 |
| POLYISOBUTENE | 0.3 |
| PEG-7 TRIMETHYLOLPROPANE COCONUT ETHER | 0.05 |
| CAFFEINE | 0.1 |
| FUCUS VESICULOSUS EXTRACT | 1 |
| FURCELLARIA LUMBRICALIS EXTRACT | 1 |
| RETINOL | 0.05 |
| PRESERVATIVES | QS |
| FRAGRANCE | QS |
| SODIUM HYDROXIDE | QS |

### Example 14

The formulation below describes a water-in-silicone cream for face, neck, eyes, decollete or body, incorporating the inventive composition which is suitable for the methods and uses according to the present invention. The percentages indicated are by weight of the composition unless stated otherwise. This example of the invention does not contain glaucine extract.

| Component Description | Weight Share |
|---|---|
| AQUA | To 100 |
| CYCLOPENTA\HEXASILOXANE | 23 |
| DIMETHICONE | 3 |
| POLYGLYCERYL-4 ISOSTEARATE | 2 |
| BUTYLENE GLYCOL | 5 |
| CETYL PEG/PPG-10/1 DIMETHICONE | 2 |
| HEXYL LAURATE | 2 |
| BIS-PEG/PPG-14/14 DIMETHICONE | 1.3 |
| DISTEARDIMONIUM HECTORITE | 1 |
| SODIUM CHLORIDE | 1 |
| CAFFEINE | 0.1 |
| RETINOL | 0.05 |
| FURCELLARIA LUMBRICALIS EXTRACT | 1 |
| FUCUS VESICULOSUS EXTRACT | 1 |
| ISOMERISED LINOLEIC ACID | 0.5 |
| FRAGRANCE | QS |
| PRESERVATIVES | QS |
| SODIUM HYDROXIDE | QS |

### Example 15

The formulation below describes an oil-in-water cream containing incorporating the inventive composition which is suitable for the methods and uses according to the present invention. The percentages indicated are by weight of the composition unless stated otherwise. This example represents a gradual tanning anti-cellulite body cream.

| COMPONENT DESCRIPTION | WEIGHT % |
|---|---|
| AQUA | To 100 |
| BUTYLENE GLYCOL | 10 |
| ISOHEXADECANE | 5 |
| GLYCERIN | 4.5 |
| DIMETHICONE | 3 |
| CYCLOPENTA\HEXASILOXANE | 3 |
| DIISOPROPYL DIMER DILINOLEATE | 2 |
| GLYCERYL STEARATE\PEG-100 STEARATE | 2.5 |
| CETYL ALCOHOL | 1 |
| STEARIC ACID | 1 |
| HYDROXYETHYL ACRYLATE/SODIUM ACRYLOYLDIMETHYL TAURATE COPOLYMER | 1 |
| BUTYROSPERMUM PARKII BUTTER | 0.5 |
| POLYISOBUTENE | 0.3 |
| XANTHAN GUM | 0.2 |
| PEG-7 TRIMETHYLOLPROPANE COCONUT ETHER | 0.05 |
| ISOMERIZED LINOLEIC ACID MIXTURE | 0.5 |
| DIHYDROXY ACETONE | 2 |
| CAFFEINE | 0.1 |
| FUCUS VESICULOSUS EXTRACT | 1 |
| FURCELLARIA LUMBRICALIS EXTRACT | 1 |
| GLAUCINE EXTRACT | 2 |
| RETINOL | 0.05 |
| PRESERVATIVES | QS |
| FRAGRANCE | QS |
| CITRIC ACID | QS |

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A topical composition for treating and/or preventing cellulite, said composition comprising:
(i) a therapeutically effective amount of each of *Furcelloria lumbricolis* and *Fucus vesiculosus,*
(ii) a therapeutically effective amount of one or more skin care actives selected from the group consisting of: glaucine, retinol, conjugated linoleic acid and caffeine; and
(iii) a dermatologically acceptable carrier.

2. A composition according to claim 1, wherein said composition comprises *Furcellaria lumbricalis* in an amount in the range 0.005% to 5%.

3. A composition according to claim 1 or 2, wherein said composition comprises *Fucus vesiculosus* in an amount in the range 0.004% to 5%.

4. A composition according to any one of claims 1 to 3, wherein said composition comprises glaucine in an amount in the range 0.002% to 5%.

5. A composition according to any preceding claim, wherein said composition comprises retinol in an amount in the range 0.01% to 2%.

6. A composition according to claim 5 wherein said composition comprises conjugated linoleic acid in an amount in the range 0.1% to 5%.

7. A composition according to claim 6 wherein said composition comprises caffeine in an amount in the range 0.05% to 0.5%.

8. A composition according to claim 1 comprising:
| | |
|---|---|
| (i) *Furcellaria lumbricalis* | 0.005 - 5 wt %; |
| (ii) *Fucus vesiculosus* | 0.004 - 5 wt %; |
| (iii) Glaucine | 0.002 - 5 wt %; and |
| a dermatologically acceptable carrier comprising: | to 100 wt %; or a composition |
|---|---|
| (i) *Furcellaria lumbricolis* | 0.005 - 5 wt%; |
| (ii) *Fucus vesiculosus* | 0.004 - 5 wt%; |
| (iii) Glaucine | 0.002 - 5 wt%; |
| (iv) Retinol | 0.01 - 2 wt%; |
| (v) Conjugated Linoleic Acid | 0.1 - 5 wt%; and |
| (vi) a dermatologically acceptable carrier | to 100 wt%. |

9. A composition according to any preceding claim, wherein the dermatologically acceptable carrier is selected from the group consisting of water, liquid or solid emollients, humectants, solvents.

10. A composition according to any preceding claim, further comprising one or more agents selected from the group consisting of fatty acids, moisturising agents and delivery enhancers.

11. A composition according to any preceding claim, further comprising one or more ingredients selected from the group consisting of: silicones, emulsifiers, thickeners, powders, film formers, rheology modifying agents and propellants.

12. A composition according to any preceding claim further comprising one or more agents selected from the group consisting of: hydroxy acids, UV filters, tanning agents and compounds which exhibit PPAR-ligand activity or anti-inflammatory activity.

13. A composition according to any preceding claim in the form of an emulsion, cream, lotion, milk, serum, concentrate, gel, body mask, body cocoon or body wrap.

14. Use of a therapeutically effective amount of each of:
(i) *Furcellaria lumbricalis* and
(ii) *Fucus vesiculosus,* together with
(iii) a therapeutically effective amount of one or more skin care actives selected from the group consisting of: glaucine, retinol, conjugated linoleic acid and caffeine; and
(iv) a dermatologically acceptable carrier, in the manufacture of a cosmetic composition for treating cellulite.

15. A cosmetic, non-therapeutic method for treating or preventing skin conditions such as cellulite, the method comprising applying to the skin the composition according to any one of claims 1 to 13.

## Patentansprüche

1. Topische Zusammensetzung zur Behandlung und/oder Verhinderung von Cellulite, wobei die Zusammensetzung umfasst:
(i) eine therapeutisch wirksame Menge von jeweils *Furcellaria lumbricalis* und *Fucus vesiculosus,*
(ii) eine therapeutisch wirksame Menge von einem oder mehreren Hautpflegewirkstoffen ausgewählt aus der Gruppe bestehend aus: Glaucin, Retinol, konjugierter Linolsäure und Koffein; und
(iii) einen dermatologisch akzeptablen Träger.

2. Zusammensetzung gemäß Anspruch 1, wobei die Zusammensetzung *Furcellaria lumbricalis* in einer Menge im Bereich von 0,005% bis 5% umfasst.

3. Zusammensetzung gemäß Anspruch 1 oder 2, wobei die Zusammensetzung *Fucus vesiculosus* in einer Menge im Bereich von 0,004% bis 5% umfasst.

4. Zusammensetzung gemäß einem der Ansprüche 1 bis 3, wobei die Zusammensetzung Glaucin in einer Menge im Bereich von 0,002% bis 5% umfasst.

5. Zusammensetzung gemäß einem vorangehenden Anspruch, wobei die Zusammensetzung Retinol in einer Menge im Bereich von 0,01% bis 2% umfasst.

6. Zusammensetzung gemäß Anspruch 5, wobei die Zusammensetzung konjugierte Linolsäure in einer Menge im Bereich von 0,1% bis 5% umfasst.

7. Zusammensetzung gemäß Anspruch 6, wobei die Zusammensetzung Koffein in einer Menge im Bereich von 0,05% bis 0,5% umfasst.

8. Zusammensetzung gemäß Anspruch 1, umfassend:
| | |
|---|---|
| (i) *Furcellaria lumbricalis* | 0,005 - 5 Gew.%; |
| *(ii) Fucus vesiculosus* | 0,004 - 5 Gew.%; |
| (iii) Glaucin | 0,002 - 5 Gew.%; und |
| einen dermatologisch akzeptablen Träger Zusammensetzung umfassend: | bis 100 Gew.%; oder eine |
|---|---|
| (i) *Furcellaria lumbricalis* | 0,005 - 5 Gew.%; |
| (ii) *Fucus vesiculosus* | 0,004 - 5 Gew.%; |
| (iii) Glaucin | 0,002 - 5 Gew.%; |
| (iv) Retinol | 0,01 - 2 Gew.%; |
| (v) konjugierte Linolsäure | 0,1 - 5 Gew.%; |
| (vi) einen dermatologisch akzeptablen Träger | bis 100 Gew.%. |

9. Zusammensetzung gemäß einem vorangehenden Anspruch, wobei der dermatologisch akzeptable Träger aus der Gruppe bestehend aus Wasser, flüssigen oder festen Emollients, Feuchthaltemitteln, Lösungsmitteln ausgewählt ist.

10. Zusammensetzung gemäß einem vorangehenden Anspruch, ferner umfassend ein oder mehrere Mittel ausgewählt aus der Gruppe bestehend aus Fettsäuren, feuchtigkeitsspendenden Mitteln und Transportverstärkern.

11. Zusammensetzung gemäß einem vorangehenden Anspruch, ferner umfassend einen oder mehrere Bestandteile ausgewählt aus der Gruppe bestehend aus:
Silikonen, Emulgatoren, Verdickungsmitteln, Pulvern, Filmbildnern, rheologiemodifizierenden Mitteln und Treibmitteln.

12. Zusammensetzung gemäß einem vorangehenden Anspruch, ferner umfassend ein oder mehrere Mittel ausgewählt aus der Gruppe bestehend aus:
Hydroxysäuren, UV-Filtern, Bräunungsmitteln und Verbindungen, die PPAR-Ligandenaktivität oder entzündungshemmende Aktivität aufweisen.

13. Zusammensetzung gemäß einem vorangehenden Anspruch in der Form einer Emulsion, Creme, Lotion, Milch, einem Serum, Konzentrat, Gel, einer Körpermaske, einem Körper-Kokon (body cocoon) oder einer Körperpackung.

14. Verwendung einer therapeutisch wirksamen Menge von jeweils:
(i) *Furcellaria lumbricalis* und
(ii) *Fucus vesiculosus,* zusammen mit
(iii) einer therapeutisch wirksamen Menge von einem oder mehreren Hautpftegewirkstoffen ausgewählt aus der Gruppe bestehend aus: Glaucin, Retinol, konjugierter Linolsäure und Koffein; und
(iv) einem dermatologisch akzeptablen Träger, bei der Herstellung einer kosmetischen Zusammensetzung zur Behandlung von Cellulite.

15. Kosmetisches, nichttherapeutisches Verfahren zur Behandlung oder Verhinderung von Hauterkrankungen wie Cellulite, wobei das Verfahren das Auftragen der Zusammensetzung gemäß einem der Ansprüche 1 bis 13 auf die Haut umfasst.

## Revendications

1. Composition topique pour traiter et/ou prévenir la cellulite, ladite composition comprenant :
(i) une quantité thérapeutiquement efficace respectivement de *Furcellaria lumbricalis* et de *Fucus vesiculosus,*
(ii) une quantité thérapeutiquement efficace d'un ou plusieurs agents actifs de soin cutané choisi(s) dans le groupe comprenant la glaucine, le rétinol, l'acide linoléique conjugué et la caféine ; et
(iii) un véhicule dermatologiquement acceptable.

2. Composition selon la revendication 1, ladite composition comprenant *Furcellaria lumbricalis* en une quantité de l'ordre de 0,005 % à 5 %.

3. Composition selon la revendication 1 ou 2, ladite composition comprenant *Furcellaria lumbricalis* en une quantité de l'ordre de 0,004 % à 5 %.

4. Composition selon l'une quelconque des revendications 1 à 3, ladite composition comprenant de la glaucine en une quantité de l'ordre de 0,002 % à 5 %.

5. Composition selon l'une quelconque des revendications précédentes, ladite composition comprenant du rétinol en une quantité de l'ordre de 0,01 % à 2 %.

6. Composition selon la revendication 5, ladite composition comprenant l'acide linoléique conjugué en une quantité de l'ordre de 0,1 % à 5 %.

7. Composition selon la revendication 6, ladite composition comprenant de la caféine en une quantité de l'ordre de 0,05 % à 0,5 %.

8. Composition selon la revendication 1, comprenant :
| | |
|---|---|
| (i) *Furcellaria lumbricalis* | 0,005 à 5 % en poids |
| (ii) *Fucus vesiculosus* | 0,004 à 5 % en poids |
| (iii) glaucine | 0,002 à 5 % en poids ; et |
| un véhicule dermatologiquement acceptable, jusqu'à 100 % en poids ; ou une composition comprenant : | |
|---|---|
| (i) *Furcellaria lumbricalis* | 0,005 à 5 % en poids |
| (ii) *Fucus vesiculosus* | 0,004 à 5 % en poids |
| (iii) glaucine | 0,002 à 5 % en poids ; |
| (iv) rétinol | 0,01 à 2 % en poids ; |
| (v) acide linoléique conjugué | 0,1 à 5 % en poids ; et |
| (vi) un véhicule dermatologiquement acceptable, jusqu'à | 100 % en poids. |

9. Composition selon l'une quelconque des revendications précédentes, dans laquelle le véhicule dermatologiquement acceptable est choisi dans le groupe comprenant l'eau, des émollients liquides ou solides, des humectants, des solvants.

10. Composition selon l'une quelconque des revendications précédentes, comprenant en outre un ou plusieurs agents choisis dans le groupe comprenant des acides gras, des agents hydratants et des agents de renforcement de la distribution.

11. Composition selon l'une quelconque des revendications précédentes, comprenant en outre un ou plusieurs ingrédients choisis dans le groupe comprenant les silicones, les émulsifiants, les épaississants, les poudres, les agents filmogènes, les agents de modification de la rhéologie et les propulseurs.

12. Composition selon l'une quelconque des revendications précédentes, comprenant en outre un ou plusieurs agents choisis dans le groupe comprenant des hydroxyacides, des filtres UV, des agents bronzants et des composés qui présentent une activité de ligand de PPAR ou une activité anti-inflammatoire.

13. Composition selon l'une quelconque des revendications précédentes, sous la forme d'une émulsion, d'une crème, d'une lotion, d'un lait, d'un sérum, d'un concentré, d'un masque corporel, d'un cocon corporel ou d'un enveloppement corporel.

14. Utilisation d'une quantité thérapeutiquement efficace de chacun des composants :
(i) *Furcellaria lumbricalis* et
(ii) *Fucus vesiculosus,* conjointement avec
(iii) une quantité thérapeutiquement efficace d'un ou plusieurs agents actifs de soin cutané choisi(s) dans le groupe comprenant la glaucine, le rétinol, l'acide linoléique conjugué et la caféine ; et
(iv) un véhicule dermatologiquement acceptable, dans la production d'une composition cosmétique pour traiter la cellulite.

15. Procédé cosmétique, non thérapeutique, pour traiter ou prévenir des affections cutanées telles que la cellulite, le procédé comprenant l'application sur la peau de la composition selon l'une quelconque des revendications 1 à 13.
